# EUROPEAN PATENT SPECIFICATION

(11) **EP 1 868 596 B1**
(45) Date of publication and mention of the grant of the patent: **15.05.2019**
(21) Application number: 06739275.3
(22) Date of filing: 22.03.2006
(51) Int. Cl.: A61K 31/201, A61K 31/202, A61K 35/74, A61P 29/00, A61K 35/745

(54) **USE OF LACTOBACILLUS RHAMNOSUS GG IN COMBINATION WITH A LONG CHAIN POLYUNSATURATED FATTY ACID FOR THE TREATMENT, PREVENTION OR REDUCTION OF SYSTEMIC INFLAMMATION IN A FORMULA-FED INFANT**
VERWENDUNG VON LACTOBACILLUS RHAMNOSUS GG IN KOMBINATION MIT EINER LANGKETTIGEN MEHRFACH UNGESÄTTIGTEN FETTSÄURE ZUR BEHANDLUNG, PRÄVENTION BZW. REDUKTION SYSTEMISCHER ENTZÜNDUNGEN BEI EINEM MIT ANFANGSMILCH ERNÄHRTEN SÄUGLING
UTILISATION DE LACTOBACILLUS RHAMNOSUS GG EN COMBINAISON AVEC UN ACIDE GRAS POLYINSATURE A CHAINE LONGUE POUR TRAITER, PREVENIR OU ATTENUER UNE INFLAMMATION SYSTEMIQUE CHEZ UN NOURRISSON NOURRI AU LAIT MATERNISE

(30) Priority: 15.04.2005 US 106794
(43) Date of publication of application: 26.12.2007
(73) Proprietor: MJN U.S. Holdings LLC, Chicago, Illinois 60606 (US)
(72) Inventor: MCMAHON, Robert, J., Evansville, Indiana 47721-0001 (US); HERZ, Udo, D-35274 Kirchhain (DE); NEU, Josef, Gainesville, Florida 32608 (US)
(74) Representative: Cawdell, Karen Teresa
(86) International application number: PCT/US2006/010415
(87) International publication number: WO 2006/113034

(56) References cited:
- WO-A-00/35443
- WO-A-98/36745
- JP-A- 9 002 959
- JP-A- 2002 332 242
- MAJAMAA H ET AL: "PROBIOTICS: A NOVEL APPROACH IN THE MANAGEMENT OF FOOD ALLERGY" JOURNAL OF ALLERGY AND CLINICAL IMMUNOLOGY, MOSBY - YEARLY BOOK, INC, US, vol. 99, no. 2, 1997, pages 179-185, XP009022566 ISSN: 0091-6749
- RUBALTELLI FIRMINO F ET AL: "Probiotics feeding prevents necrotizing enterocolitis in preterm infants: A prospective double-blind study" PEDIATRIC RESEARCH, vol. 47, no. 4 Part 2, April 2000 (2000-04), page 346A, XP009069417 & JOINT MEETING OF THE PEDIATRIC ACADEMIC SOCIETIES AND THE AMERICAN ACADEMY OF PEDIATRICS.; BOSTON, MASSACHUSETTS, USA; MAY 12-16, 2000 ISSN: 0031-3998
- SHERMAN MICHAEL P ET AL: "Neonatal small bowel epithelia: enhancing anti-bacterial defense with lactoferrin and Lactobacillus GG" BIOMETALS, vol. 17, no. 3, June 2004 (2004-06), pages 285-289, XP002390229 ISSN: 0966-0844
- CARLSON S E ET AL: "LOWER INCIDENCE OF NECROTIZING ENTEROCOLITIS IN INFANTS FED A PRETERM FORMULA WITH EGG PHOSPHOLIPIDS" PEDIATRIC RESEARCH, WILLIAMS AND WILKINS, BALTIMORE, MD,, US, vol. 44, no. 4, October 1998 (1998-10), pages 491-498, XP000892677 ISSN: 0031-3998
- MIHRSHAHI SEEMA ET AL: "Eighteen-month outcomes of house dust mite avoidance and dietary fatty acid modification in the Childhood Asthma Prevention Study (CAPS)." JOURNAL OF ALLERGY AND CLINICAL IMMUNOLOGY, vol. 111, no. 1, January 2003 (2003-01), pages 162-168, XP002391427 ISSN: 0091-6749
- NAGAKURA T ET AL: "Dietary supplementation with fish oil rich in omega-3 polyunsaturated fatty acids in children with bronchial asthma" EUROPEAN RESPIRATORY JOURNAL, vol. 16, no. 5, November 2000 (2000-11), pages 861-865, XP002391428 ISSN: 0903-1936
- DAS UNDURTI N: "A perinatal strategy to prevent coronary heart disease." NUTRITION (BURBANK, LOS ANGELES COUNTY, CALIF.) 2003 NOV-DEC, vol. 19, no. 11-12, November 2003 (2003-11), pages 1022-1027, XP002391429 ISSN: 0899-9007
- KAILA M ET AL: "FATTY ACIDS IN SUBSTITUTE FORMULAS FOR COW'S MILK ALLERGY" ALLERGY, MUNSKGAARD, COPENHAGEN, DK, vol. 54, no. 1, 1999, pages 74-77, XP001146262 ISSN: 0105-4538
- PENA JEREMY ANDREW ET AL: "Lactobacillus Rhamnosus GG decreases TNF-alpha production in lipopolysaccharide-activated murine macrophages by a contact-independent mechanism" CELLULAR MICROBIOLOGY, BLACKWELL SCIENCE, OXFORD,, GB, vol. 5, no. 4, April 2003 (2003-04), pages 277-285, XP002970546 ISSN: 1462-5814
- SPOONER C E ET AL: "The role of tumor necrosis factor in sepsis", CLINICAL IMMUNOLOGY AND IMMUNOPATHOLOGY, SAN DIEGO, CA, US, vol. 62, no. 1, 1 January 1992 (1992-01-01), pages S11-S17, XP026018052, ISSN: 0090-1229, DOI: 10.1016/0090-1229(92)90036-N [retrieved on 1992-01-01]
- SEITZ ET AL: "Protective effect of tumor necrosis factor @a antibody on experimental necrotizing enterocolitis in the rat", JOURNAL OF PEDIATRIC SURG, W. B. SAUNDERS COMPANY, US, vol. 40, no. 9, 1 September 2005 (2005-09-01), pages 1440-1445, XP005075566, ISSN: 0022-3468, DOI: 10.1016/J.JPEDSURG.2005.05.043

## Description

### BACKGROUND OF THE INVENTION

### (1) Field of the Invention

The present invention relates generally to a use of a probiotic in combination with at least one long chain polyunsaturated fatty acid in the manufacture of a medicament for the treatment, prevention, or reduction of systemic inflammation in a formula-fed infant.

### (2) Description of the Related Art

The inflammatory response is an attempt by the body to restore and maintain homeostasis after invasion by an infectious agent, antigen challenge, or physical, chemical or traumatic damage. Localized inflammation is contained in a specific region and can exhibit varying symptoms, including redness, swelling, heat and pain.

While the inflammatory response is generally considered a healthy response to injury, the immune system can present an undesirable physiological response if it is not appropriately regulated. In these situations, the body's normally protective immune system causes damage to its own tissue by treating healthy tissue as if it is infected or somehow abnormal. Alternatively, if there is an injury, the inflammatory response may be out of proportion with the threat it is dealing with. This inflammatory response can cause more damage to the body than the agent itself would have produced.

The inflammatory response has been found in part to consist of an increased expression of both pro-inflammatory and anti-inflammatory cytokines. Cytokines are low molecular weight, biologically active proteins involved in the coordination of immunological and inflammatory responses and communication between specific immune cell populations. A number of such cell types produce cytokines, including neutrophils, monocytes, and lymphocytes as the major sources during inflammatory reactions due to their large numbers at the site of injury.

Multiple mechanisms exist by which cytokines generated at inflammatory sites influence the inflammatory response. If a pro-inflammatory response is not successfully countered by anti-inflammatory cytokines, however, uncontrolled systemic inflammation can occur.

In contrast to localized inflammation, systemic inflammation is widespread throughout the body. This type of inflammation may include localized inflammation at specific sites, but may also be associated with general "flu-like" symptoms, including fever, chills, fatigue or loss of energy, headaches, loss of appetite, and muscle stiffness. Systemic inflammation can lead to protein degradation, catabolism and hypermetabolism. As a consequence, the structure and function of essential organs, such as muscle, heart, immune system and liver may be compromised and can contribute to multi-organ failure and mortality. Jeschke, et al., Insulin Attenuates the Systemic Inflammatory Response to Thermal Trauma, Mol. Med. 8(8):443-450 (2002). Although enormous progress has been achieved in understanding the mechanisms of systemic inflammation, the mortality rate due to this disorder remains unacceptably high.

Often, whether the cytokine response is pro- or anti-inflammatory depends on the balance of individual microorganisms that colonize the intestinal lumen at any particular time. It is well known that the mucosal surface of the intestinal tract is colonized by an enormously large, complex, and dynamic collection of microorganisms. The composition of the intestinal microflora varies along the digestive tract as well as in different micro-habitats, such as the epithelial mucus layer, the deep mucus layer of the crypts, and the surface of mucosal epithelial cells. The specific colonization depends on external and internal factors, including luminally available molecules, mucus quality, and host-microbial and microbial-microbial interactions. Murch, S.H., Toll of Allergy Reduced by Probiotics, Lancet, 357:1057-1059 (2001).

These microorganisms, which make up the gut microflora, are actively involved with the immune response. They interact with the epithelium in conditions of mutual beneficial relationships for both partners (symbiosis) or in conditions of benefit for one partner, without being detrimental to the other (commensalisms). Hooper, et al., How Host-Microbial Interactions Shape the Nutrient Environment of the Mammalian Intestine, Annu. Rev. Nutr. 22:283-307 (2002). In fact, considerable evidence is emerging which shows a strong interplay or "cross-talk" between the intestinal microflora and the diverse population of cells in the intestinal mucosa. Bourlioux, et al., The Intestine and its Microflora are Partners for the Protection of the Host: Report on the Danone Symposium "The Intelligent Intestine," held in Paris, June 14, 2002, Am. J. Clin. Nutr. 78:675 (2003); Hooper, L.V. & Gordon, J.I., Commensal Host-Bacterial Relationships in the Gut, Sci. 292:1115 (2001); Haller, et al., Non-Pathogenic Bacteria Elicit a Differential Cytokine Response by Intestinal Epithelial Cell/Leucocyte Co-Cultures, GUT 47:79 (2000); Walker, W.A., Role of Nutrients and Bacterial Colonization in the Development of Intestinal Host Defense, J. Pediatr. Gastroenterol. Nutr. 30:S2 (2000). Additionally, the gut microflora has been shown to elicit specific immune responses at both a local and systemic level in adults. Isolauri, E., et al., Probiotics: Effects on Immunity, Am. J. Clin. Nutr. 73:444S-50S (2001).

The gut microflora in infants is well known to be far less developed than that of an adult. While the microflora of the adult human consists of more than 10¹³ microorganisms and nearly 500 species, some being harmful and some being beneficial, the microflora of an infant contains only a fraction of those microorganisms, both in absolute number but also species diversity. Infants are born with a sterile gut, but acquire intestinal flora from the birth canal, their initial environment, and what they ingest. Because the gut microflora population is very unstable in early neonatal life, it is often difficult for the infant's gut to maintain the delicate balance between harmful and beneficial bacteria, thus reducing the ability of the immune system to function normally.

It is especially difficult for formula-fed infants to maintain this balance due to the differences between the bacterial species in the gut of a formula-fed and breast-fed infant. The stool of breast-fed infants contains predominantly *Bifidobacterium,* with *Streptococcus* and *Lactobacillus* as less common contributors. In contrast, the microflora of formula-fed infants is more diverse, containing *Bifidobacterium* and *Bacteroides* as well as the more pathogenic species, *Staphylococcus, Escherichia coli,* and *Clostridia.* The varied species of *Bifidobacterium* in the stools of breast-fed and formula-fed infants differ as well. A variety of factors have been proposed as the cause for the different fecal flora of breast-fed and formula-fed infants, including the lower content and different composition of proteins in human milk, a lower phosphorus content in human milk, the large variety of oligosaccharides in human milk, and numerous humoral and cellular mediators of immunologic function in breast milk. Agostoni, et al., Probiotic Bacteria in Dietetic Products for Infants: A Commentary by the ESPGHAN Committee on Nutrition, J. Pediatr. Gastro. Nutr. 38:365-374 (Apr. 2004).

Because the microflora of formula-fed infants is so unstable and the gut microflora largely participate in stimulation of gut immunity, formula-fed infants are more likely to develop inflammatory illnesses. Many of the major illnesses that affect infants, including chronic lung disease, periventricular leukomalacia, neonatal meningitis, neonatal hepatitis, sepsis, and necrotizing enterocolitis are inflammatory in nature. Depending on the particular disease, the accompanying inflammation can occur in a specific organ, such as the lung, brain, liver or intestine, or the inflammation can truly be systemic in nature.

For example, chronic lung disease causes the tissues inside the lungs to become inflamed while neonatal meningitis involves inflammation of the linings of the brain and spinal cord. Periventricular leukomalacia is caused by inflammatory damage to the periventricular area in the developing brain. Necrotizing enterocolitis causes inflammation in the intestine that may result in destruction of part or all of the intestine and neonatal hepatitis involves an inflammation of the liver that occurs in early infancy. Sepsis, also known as systemic inflammatory response syndrome, is a severe illness caused by an overwheming infection of the bloodstream by toxin-producing bacteria, where the presence of pathogens in the bloodstream elicit an inflammatory response throughout the entire body.

Premature and critically ill infants also represent a serious challenge in terms of developing gut immunity and preventing systemic inflammation. Preterm or critically ill infants are often placed immediately into sterile incubators, where they remain unexposed to the bacterial populations to which a healthy, term infant would normally be exposed. This may delay or impair the natural colonization process. These infants are also often treated with broad-spectrum antibiotics, which kill commensal bacteria that attempt to colonize the infant's intestinal tract. Additionally, these infants are often nourished by means of an infant formula, rather than mother's milk. Each of these factors may cause the infant's gut microflora to develop improperly, thus causing or precipitating life-threatening systemic inflammation.

One way to encourage gut colonization with beneficial microorganisms in formula-fed infants is through the administration of probiotic bacteria. Probiotic bacteria are living microorganisms that exert beneficial effects on the health of the host. *Lactobacillus* spp. and *Bifidobacterium* spp., which are normal inhabitants of the healthy intestine, are common species of probiotics.

Unfortunately, there are very few published studies on the clinical effects of probiotic supplementation on infants. Agostoni, C., et al., Probiotic Bacteria in Dietetic Products for Infants: A Commentary by the ESPGHAN Committee on Nutrition, J. Pediatr. Gastro. Nutr. 38:365-374 (2004). Even less is known about the capability of probiotics to regulate intestinal inflammation and alter the propagation of the inflammatory response to other organs in infants.

Results from studies regarding the effects of probiotics on infants are controversial. For example, a 1994 study concluded that the administration of standard infant formula supplemented with *Bifidobacterium lactis* and *Streptococcus thermophilus* reduced the prevalence of nosocomical diarrhea compared with placebo. Saavedra, J., et al., Feeding of Bifidobacterium bifidum and Streptococcus thermophilus to Infants in Hospital for Prevention of Diarrhea and Shedding of Rotavirus, Lancet 344:1049-49 (1994). In contrast, however, a 1999 study reported no protective effect of infant formula supplemented with *Bifidobacterium* alone or in combination with *S*. *thermophilus* on episodes of diarrhea. Phuapradit, P., et al., Reduction of Rotavirus Infection in Children Receiving Bifidobacteria-Supplemented Formula, J. Med. Assoc. Thai. 82:S43-48 (1999).

U.S. Patent App. No. 20040208863 to Versalovic, et al. is directed to a compound which has anti-inflammatory activity and is secreted from lactic acid bacteria. The application describes the use of *Lactobacillus rhamnosus* GG (LGG) to inhibit pro-inflammatory cytokine production. The reference, however, focuses on adult models and does not disclose or suggest that the invention would be beneficial for infants. As explained above, the gut and immune system of an infant is very unlike that of an adult. Because the bacterial populations and species vary so immensely between the gut of an infant and adult, and the large difference in maturity of the immune system in these two populations, it cannot be assumed that the same result would be achieved in an infant.

U.S. Patent App. No. 20040147010 to Vidal, et al. relates to a method for reducing or preventing inflammatory processes associated with bacterially-mediated disease in the GI tract, bone, skin, eye, ear, lung and oral cavity of a human. The method comprises administering an effective amount of lipoteichoic acid (LTA) from lactic acid bacteria and/or administering a lactic acid bacteria that produces LTA. The application also notes that these compositions could modify bacterial colonization and infection during the neonatal period.

The bacterial strains of Vidal's application were *Lactobacillus acidophilus* and *Lactobacillus johnsonii.* Vidal did not indicate the use of LGG. In fact, Vidal discloses that "LTAs from Gram-positive bacteria show great diversity from one bacterial strain to another." Vidal app., p. [0006]. Therefore, it should not be assumed that merely because *L. acidophilus* and *L. johnsonii* caused an anti-inflammatory effect in the adult colonic cell line that was assayed, that all *Lactobacillus* species would.

Vidal additionally notes that LTA from certain species of bacteria mediate a pro-inflammatory effect rather than an anti-inflammatory effect on immune cells. Vidal app., p. [0005]. Thus, because LTA can be pro-inflammatory or anti-inflammatory, depending on the bacterial species, Vidal's disclosure is limited to the species specifically described. As Vidal has recognized in a published article, "the biological activity of LTAs [of different bacterial species] cannot be predicted." Vidal, et al., Lipoteichoic Acids from Lactobacillus johnsonii Strain La1 and Lactobacillus acidophilus Strain La10 Antagonize the Responsiveness of Human Intestinal Epithelial HT29 Cells to Lipopolysaccharide and Gram-Negative Bacteria, Infect. Immun. 70:2057-2064 (2002).

A reference directed to the use of probiotics is Majamaa H et. al. Probiotics: A novel approach in the management of food allergy" Journal of Allergy and Clinical immunology, Mosby - Yearly Book, inc, US, vol. 99, no 2, 1997, pages 179-185.

A reference directed to the benefits of LGG with neonatals is Sherman Michael P. et al. "Neonatal small bowel epithelia: enhancing antibacterial defense with lactoferrin and Lactobaciluus GG" Biometals, vol. 17, no. 3, June 2004, pages 285-289.

Based on the above references, the effect of LGG on the infant immune system has not heretofore been disclosed. There are large and fundamental differences between the infant gut and immune system compared to those of an adult. Therefore, studies that focus on adult subjects or adult cell lines are not useful in evaluating the effect of LGG on infants. It has not previously been shown that LGG exhibits a systemic immune effect on formula-fed infants. In addition, it has not been shown that LGG supplementation in formula-fed infants would prevent or reduce systemic inflammation to a level similar to that of a breast-fed infant. Accordingly, it would be beneficial to provide a method for reducing or preventing systemic inflammation in formula-fed infants comprising the administration of LGG.

### SUMMARY OF THE INVENTION

The invention is defined in the claims. Briefly, therefore, the present invention is directed to a novel use of LGG in combination with at least one long chain polyunsaturated fatty acid (LCPUFA) in the manufacture of a medicament for the treatment, prevention, or reduction of systemic inflammation in a formula-fed infant.

### BRIEF DESCRIPTION OF THE DRAWINGS

For a more complete understanding of the present invention, reference is now made to the following descriptions taken in conjunction with the accompanying drawings.
Figure 1 illustrates the effect of LGG on rat pup growth, expressed as body weight over the time course of the study.
Figure 2 illustrates the effect of LGG on the intestinal morphology of the rat pups, as depicted by micrographs of the intestinal tissue under conditions of inflammation with or without the administration of LGG.
Figure 3 illustrates the effect of LGG on cytokine induced neutrophil chemoattractant-1 (CINC-1) peptide production from the intestine (Fig. A), liver (Fig. B), plasma (Fig. C) and lung (Fig. D) using enzyme-linked immunosorbent assay (ELISA).
Figure 4 illustrates the effect of LGG on TNF-α production from plasma (Fig. A) and lung (Fig. B) using ELISA.
Figure 5 illustrates the effect of LGG on intestinal MPO activity from distal small intestine (Fig. A) and lung (Fig. B).
Figure 6 illustrates the effect of LGG on cytokine abundances. Figure A shows cytokine levels in the lung, Figure B shows cytokine levels in the liver, and Figure C shows cytokine levels in the plasma.

### DETAILED DESCRIPTION OF THE PREFERRED EMBODIMENTS

Reference now will be made in detail to the embodiments of the invention, one or more examples of which are set forth below. Each example is provided by way of explanation of the invention.

It is to be understood by one of ordinary skill in the art that the present discussion is a description of exemplary embodiments.

### Abbreviations

As used herein, the following abbreviations are used: LGG, *Lactobacillus rhamnosus* GG; LCPUFA, long-chain polyunsaturated fatty acid; LPS, lipopolysaccharide; IL, interleukin; TNF, tumor necrosis factor; CINC-1, cytokine induced neutrophil chemoattractant-1; GRO/KC, growth-related oncogene; ELISA, enzyme-linked immunosorbent assay; RT-PCR, reverse transcription-polymerase chain reaction; ANOVA, analysis of variance; SD, standard deviation; PAF, platelet-activating factor; RMS, rat milk substitute; MPO, myloperioxidase; TLRs, Toll-like receptors; EPA, eicosapentaenoic acid; DHA, docosahexaenoic acid; ARA, arachidonic acid.

### Definitions

The term "probiotic" means a microorganism that exerts beneficial effects on the health of the host.

The term "prebiotic" means a non-digestible food ingredient that stimulates the growth and/or activity of probiotics.

As used herein, the term "treating" means ameliorating, improving or remedying a disease, disorder, or symptom of a disease or condition.

The term "reducing" means to diminish in extent, amount, or degree.

The term "preventing" means to stop or hinder a disease, disorder, or symptom of a disease or condition through some action.

The term "systemic", as used herein, means relating to or affecting the entire body.

The terms "therapeutically effective amount" refer to an amount that results in an improvement or remediation of the disease, disorder, or symptoms of the disease or condition.

The term "preterm" means an infant born before the end of the 37th week of gestation.

The term "infant" means a human that is less than about 1 year old.

As used herein, the term "infant formula" means a composition that satisfies the nutrient requirements of an infant by being a substitute for human milk. In the United States, the contents of an infant formula is dictated by the federal regulations set forth at 21 C.F.R. Sections 100, 106, and 107. These regulations define macronutrient, vitamin, mineral, and other ingredient levels in an effort to stimulate the nutritional and other properties of human breast milk.

### Invention

In accordance with the present invention, a novel use of LGG in combination with at least one long chain polyunsaturated fatty acid (LCPUFA) in the manufacture of a medicament for the treatment or prevention of systemic inflammation in a formula-fed infant has been discovered.

LGG is a probiotic strain isolated from healthy human intestinal flora. It was disclosed in U.S. Patent No. 5,032,399 to Gorbach, et al.*,* which is herein incorporated in its entirety, by reference thereto. LGG is resistant to most antibiotics, stable in the presence of acid and bile, and attaches avidly to mucosal cells of the human intestinal tract. It survives for 1-3 days in most individuals and up to 7 days in 30% of subjects. In addition to its colonization ability, LGG also beneficially affects mucosal immune responses. LGG is deposited with the depository authority American Type Culture Collection under accession number ATCC 53103.

In the use of the invention, a therapeutically effective amount of LGG may correspond to between about 1x10⁴ and 1x10¹² cfu/L/kg/day for an infant. In another embodiment, the present invention comprises the administration of between about 1x10⁶ and 1x10⁹ cfu/L/kg/day LGG to an infant. In yet another embodiment, the present invention comprises the administration of about 1x10⁸ cfu/L/kg/day LGG to an infant.

Suitable LCPUFAs useful in the present invention may include, but are not limited to, α-linoleic acid, γ-linoleic acid, linoleic acid, linolenic acid, eicosapentanoic acid (EPA), ARA and DHA. In the use of the present invention, an effective amount of LCPUFA may correspond to between 3 mg per kg of body weight per day to 150 mg per kg of body weight per day. In one embodiment of the invention, the amount is from 6 mg per kg of body weight per day to 100 mg per kg of body weight per day. In another embodiment the amount is from 10 mg per kg of body weight per day to 60 mg per kg of body weight per day.

The form of administration of LGG and LCPUFA in the use of the invention is not critical, as long as a therapeutically effective amount of LGG is administered in combination with at least one LCPUFA. Most conveniently, the LGG and LCPUFA are supplemented into infant formula which is then fed to an infant.

In an embodiment, the infant formula for use in the present invention is nutritionally complete and contains suitable types and amounts of lipid, carbohydrate, protein, vitamins and minerals. The amount of lipid or fat typically can vary from 3 to 7 g/100 kcal. The amount of protein typically can vary from 1 to 5 g/100 kcal. The amount of carbohydrate typically can vary from 8 to 12 g/100 kcal. Protein sources can be any used in the art, e.g., nonfat milk, whey protein, casein, soy protein, hydrolyzed protein, amino acids, and the like. Carbohydrate sources can be any used in the art, e.g., lactose, glucose, corn syrup solids, maltodextrins, sucrose, starch, rice syrup solids, and the like. Lipid sources can be any used in the art, e.g., vegetable oils such as palm oil, soybean oil, palmolein, coconut oil, medium chain triglyceride oil, high oleic sunflower oil, high oleic safflower oil, and the like.

Conveniently, commercially available infant formula can be used. For example, Enfamil®, Enfamil® Premature Formula, Enfamil® with Iron, Lactofree®, Nutramigen®, Pregestimil®, and ProSobee® (available from Mead Johnson & Company, Evansville, IN, U.S.A.) may be supplemented with suitable levels of LGG and LCPUFA and used in practice of the invention.

In one embodiment of the invention, LGG and LCPUFA can be combined with one or more additional probiotics to treat or prevent systemic inflammation in formula-fed infants. Any probiotic known in the art will be acceptable in this embodiment. In a particular embodiment, the probiotic is chosen from the group consisting of *Lactobacillus* and *Bifidobacterium.*

In another embodiment of the invention, LGG and LCPUFA can be combined with one or more prebiotics to treat or prevent systemic inflammation in formula-fed infants. Any prebiotic known in the art will be acceptable in this embodiment. Prebiotics of the present invention may include lactulose, galacto-oligosaccharide, fructo-oligosaccharide, isomalto-oligosaccharide, soybean oligosaccharides, lactosucrose, xylo-oligosacchairde, and gentio-oligosaccharides.

In an embodiment, LGG is administered in combination with DHA. In another embodiment, LGG is administered in combination with ARA. In yet another embodiment, LGG is administered in combination with both DHA and ARA. Commercially available infant formula that contains DHA, ARA, or a combination thereof may be supplemented with LGG and used in the present invention. For example, Enfamil® LIPIL®, which contains effective levels of DHA and ARA, is commercially available and may be supplemented with LGG and utilized in the present invention.

In one embodiment, both DHA and ARA are used in combination with LGG to treat systemic inflammation in infants. In this embodiment, the weight ratio of ARA:DHA is typically from 1:3 to 9:1. In one embodiment of the present invention, this ratio is from 1:2 to 4:1. In yet another embodiment, the ratio is from 2:3 to 2:1. In one particular embodiment the ratio is about 2:1.

The effective amount of DHA in an embodiment of the present invention is typically from 3 mg per kg of body weight per day to 150 mg per kg of body weight per day. In one embodiment of the invention, the amount is from 6 mg per kg of body weight per day to 100 mg per kg of body weight per day. In another embodiment the amount is from 10 mg per kg of body weight per day to 60 mg per kg of body weight per day. In yet another embodiment the amount is from 15 mg per kg of body weight per day to 30 mg per kg of body weight per day.

The effective amount of ARA in an embodiment of the present invention is typically from 5 mg per kg of body weight per day to 150 mg per kg of body weight per day. In one embodiment of this invention, the amount varies from 10 mg per kg of body weight per day to 120 mg per kg of body weight per day. In another embodiment, the amount varies from 15 mg per kg of body weight per day to 90 mg per kg of body weight per day. In yet another embodiment, the amount varies from 20 mg per kg of body weight per day to 60 mg per kg of body weight per day.

The amount of DHA in infant formulas for use with the present invention typically varies from 5 mg/100 kcal to 80 mg/100 kcal. In one embodiment of the present invention it varies from 10 mg/100 kcal to 50 mg/100 kcal; and in another embodiment from 15 mg/100 kcal to 20 mg/100 kcal. In a particular embodiment of the present invention, the amount of DHA is about 17 mg/100 kcal.

The amount of ARA in infant formulas for use with the present invention typically varies from 10 mg/100 kcal to 100 mg/100 kcal. In one embodiment of the present invention, the amount of ARA varies from 15 mg/100 kcal to 70 mg/100 kcal. In another embodiment the amount of ARA varies from 20 mg/100 kcal to 40 mg/100 kcal. In a particular embodiment of the present invention, the amount of ARA is about 34 mg/100 kcal.

The infant formula supplemented with oils containing DHA and ARA for use with the present invention can be made using standard techniques known in the art. For example, they can be added to the formula by replacing an equivalent amount of an oil, such as high oleic sunflower oil, normally present in the formula. As another example, the oils containing DHA and ARA can be added to the formula by replacing an equivalent amount of the rest of the overall fat blend normally present in the formula without DHA and ARA.

The source of DHA and ARA can be any source known in the art. In an embodiment of the present invention, sources of DHA and ARA are single cell oils as taught in U.S. Pat. Nos. 5,374,567; 5,550,156; and 5,397,591. However, the present invention is not limited to only such oils. DHA and ARA can be in natural or refined form.

In one embodiment, the source is substantially free of EPA. For example, in one embodiment of the present invention the infant formula contains less than about 16 mg EPA/100 kcal; in another embodiment less than about 10 mg EPA/100 kcal; and in yet another embodiment less than about 5 mg EPA/100 kcal. One particular embodiment contains substantially no EPA. Another embodiment is free of EPA in that even trace amounts of EPA are absent from the formula.

It is believed that provision of the combination of LGG with DHA and/or ARA provides complimentary or synergistic effects with regards to the anti-inflammatory properties of formulations containing these agents. While not wishing to be tied to this or any other theory, probiotics such as LGG are thought to impart anti-inflammatory effects in part through interaction with specific receptors, known as Toll-like receptors (TLRs) on the surface of specific immune cells. Direct or indirect interaction between LGG and these receptors initiates an intracellular signal transduction cascade that results in the alteration of gene expression in these target cells. It is this specific interaction and resulting alteration in gene expression and other cellular effects that is thought to be involved in the modulation of inflammation.

In contrast, ω-3 fatty acids such as DHA are thought to impart anti-inflammatory action through altering the production of pro-inflammatory, fatty acid derived, mediators broadly known as eicosanoids. ω-6 fatty acids, such as ARA, which are located in the phospholipid pool of cell membranes, are released during the inflammatory response and liberate a pool of free ARA. This pool of ARA is then acted upon by two classes of enzymes, known as lipoxygenases and cyclooxygenases, which produce a specific spectrum of eicosanoids including the 2-series prostanoids, such as prostaglandins, thromboxanes, and leukotrienes. These eicosanoids are known to have a plethora of pro-inflammatory actions in many cell types and organs. It is known that diets rich in ω-3 fatty acids, such as EPA and DHA, are competitors for ω-6 fatty acids in several steps of this process and, therefore, moderate the pro-inflammatory effects of ARA. For example, ω-3 fatty acids modulate the elongation of the ω-6 fatty acids into ARA, the incorporation of ARA into the cell membrane phospholipid pool, and the production of pro-inflammatory eicosanoids from ARA. The combination of DHA and ARA, therefore, provides distinct, but complimentary, actions to moderate the inflammatory response in multiple tissues.

As an alternative to an infant formula, the LGG and LCPUFA can be administered as a supplement not integral to the formula feeding. For example, LGG can be ingested in the form of a pill, tablet, capsule, caplet, powder, liquid or gel. In this embodiment of the invention, an LGG supplement can be ingested in combination with other nutrient supplements, such as vitamins, or in combination with a LCPUFA supplement, such as DHA or ARA.

In another embodiment, the LGG and/or LCPUFA are encapsulated in a sugar, fat, or polysaccharide matrix to further increase the probability of bacterial survival. Compositions of the present invention can also be provided in a form suitable for infants selected from the group consisting of follow-on formula, beverage, milk, yogurt, fruit juice, fruit-based drink, chewable tablet, cookie, cracker, or a combination thereof.

In the present invention, the infant is formula-fed. In one embodiment the infant is formula-fed from birth. In another embodiment, the infant is breast-fed from birth until an age which is less than one year, and is formula-fed thereafter, at which time LGG and LCPUFA supplementation begins.

In a particular embodiment of the present invention, the use comprises treating or preventing systemic inflammation in a formula-fed preterm infant. In this use, LGG and LCPUFA can be administered to the preterm infant in the form of an infant formula or any other suitable form. In another embodiment, LGG can be administered to the preterm infant in combination with DHA and/or ARA to create a potentially synergistic anti-inflammatory effect.

In a particular use of the present invention, the administration of LGG and LCPUFA reduces or prevents the systemic release of one or more pro-inflammatory cytokines or chemokines in a formula-fed infant. As used herein, "pro-inflammatory" cytokines or chemokines include those known in the art to be involved in the up-regulation of inflammatory reactions. Examples include, but are not limited to TNF-α, IL-1β, IL-6, IL-18, and GRO/KC.

Chemokines are a group of cytokines that enable the migration of leukocytes from the blood to the tissues at the site of inflammation. When produced in excess amounts, chemokines can lead to damage of healthy tissue. Growth-related oncogene (GRO/KC) is a chemokine which recruits immune cells to the site of inflammation. It is the human counterpart to rat cytokine-induced neutrophil chemoattractant (CINC-1), and is functionally related to the interleukin-8 family.

LGG and LCPUFA reduce or prevent the systemic release of MPO in a formula-fed infant. MPO is an iron-containing protein located in the azurophilic granules of neutrophils and in the lysosomes of monocytes. It uses hydrogen peroxidase to convert chloride to hypochlorous acid. The produced hypochlorous acid then reacts with and destroys bacteria. During inflammation, MPO levels peak as it attempts to destroy pathogens. Thus, the enzyme is extremely useful as a marker of inflammation. Frode, T. & Medeiros, Y. Myloperoxidase and Adenosine-Deaminase Levels in the Pleural Fluid Leakage Induced by Carrageenan in the Mouse Model of Pleurisy, MI 10:4, 223-227 (2001).

As will be seen in the examples, LGG has been shown to reduce systemic inflammation in a formula-fed infant to a level similar to that of breast-fed infants. Physical damage in the intestinal mucosa of formula-fed rat infants was reduced to a level similar to that of mother's milk-fed rat infants when their diets were supplemented with LGG. Additionally, CINC-1, MPO, and various cytokine levels in the formula-fed rat infants were reduced to levels similar to that of mother's milk fed rat infants when supplemented with LGG.

The following examples describe various embodiments of the present invention. Other embodiments within the scope of the claims herein will be apparent to one skilled in the art from consideration of the specification or practice of the invention as disclosed herein. It is intended that the specification, together with the examples, be considered to be exemplary only, with the scope of the invention being indicated by the claims which follow the examples. In the examples, all percentages are given on a weight basis unless otherwise indicated.

### Example 1

This example describes the materials and methods necessary to show the effect of LGG on formula-fed neonatal rat pups. In two separate experiments, ten Sprague-Dawley (Taconic, Germantown, NY) infant rats were randomly assigned to two gastrostomy feeding groups with five rats per group. Gastrostomy feeding, using the rat infant "pup-in-the-cup" model, began on day 7 of life of the rat pups. The gastrostomy feeding tubes were constructed from 14-cm sections of polyethylene tubing that were inserted into the stomach of the pups. This is a commonly used model in studies of developmental nutrition when it is important to manipulate nutritional composition in the absence of maternal feedings. The gastrostomy placement was done under isoflurane anesthesia. Timer-controlled syringe pumps were connected to the feeding tubes and were set to feed the rats for the first 20 minutes of every hour at a weight-dependent flow rate. Five mother-reared rats of the same age were used as reference controls.

During a 2-day acclimation period, the gastrostomy-fed rat pups were fed with rat milk substitute (RMS). The protein component of the RMS was between 30 and 40 g/kg/day, which is similar to that of mother's milk and is required for normal growth. One of the RMS fed groups was also given a supplement of 1x10⁸ cfu/L/kg/day LGG. The other group was fed RMS alone, without LGG supplementation. All of the gastrostomy-fed groups received the same quantity of fat and carbohydrates.

Lipopolysaccharide (LPS) from *Escherichia coli* 0127:B8 (LPS; Sigma, St. Louis, MO) was dissolved in water by vortexing at a concentration of 2 mg/ml. The gastrostomy-fed rats were given between 0.25 and 0.5 mg/kg/day of LPS via the gastrostomy tube starting 2 days after the initiation of artificial feeding. The pups were given LPS supplementation for 6 days. This dose was determined in pilot studies to result in occasional shivering, piloerection, and poor weight gain but was not associated with a significant increase in mortality over a 6-day period.

At the end of the 6-day treatment period, the rat pups were euthanized with an overdose of pentobarbital sodium. The small intestine was removed and separated into three parts: the ileum, jejunum, and duodenum, stored at -80°C for enzyme assays and ELISA, or fixed in 10% neutral buffered formalin for intestinal morphology. Lung, liver and plasma were stored at -80°C for enzyme assays and ELISA.

Sigmastat statistical software (SPSS, Chicago, IL) was used to analyze body weight, villus measurements, and enzyme activities, MPO, ELISA for CINC-1, and TNF-α and densitometry results for RT-PCR. All data were reported as means ± standard deviation (SD). A one-way analysis of variance between groups (ANOVA) was used to determine whether a significant difference was present among all treatment groups.

### Example 2

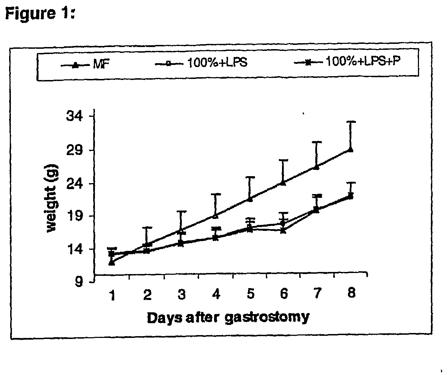

This example illustrates the effect of LGG on the growth of pups after gastrostomy feeding. The rat pups were weighed daily after the gastrostomy feeding and compared to mother-fed reference animals. Figure 1 shows that mother-fed animals grew more rapidly than the LPS-treated, gastrostomy-fed pups. Line graphs represent the increased rate of pup body weight with the time expressed increase from the beginning of the study. Providing LGG to gastrostomy-fed, LPS treated pups did not improve weight gain.

### Example 3

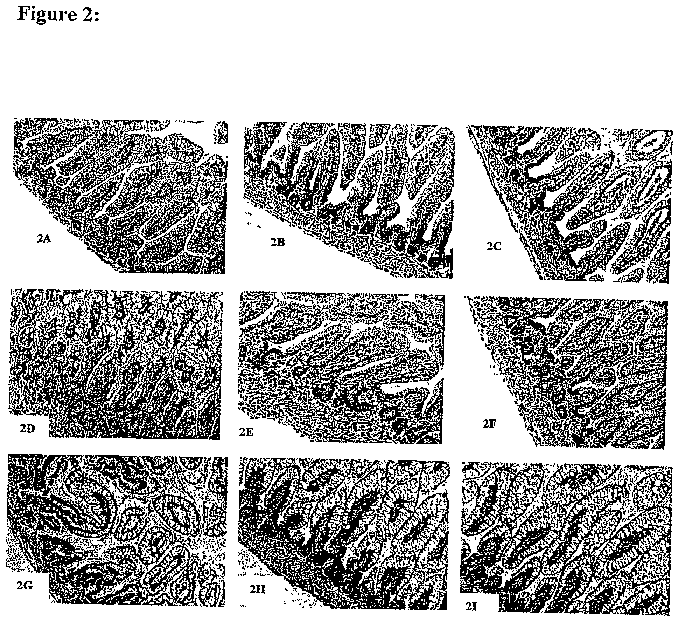

This example illustrates the effect of LGG on the intestinal morphology of the rat pups. The microscopy studies were focused on the ileum because this is a region that is most highly susceptible to certain pathologies in infants (e.g., necrotizing enterocolitis and nonnecrotizing enterocolitis-related perforations). Formalin-fixed ileum samples were embedded in paraffin; 6-µm sections were cut using a 2030 Reichert-Jung paraffin microtome. The sections were then stained with a routine hematoxylin and eosin (H&E) stain. Figure 2 shows the results of this stain.

Sections of ileum from LPS-treated rat pups (Figs. 2G-2I) showed a striking metaplasia in the villous epithelium, with increased clearing of the cytoplasm, compared to the mother-reared controls (Figs. 2A-2C). Figures 2G-2F show that these sections also featured expansion of the lamina propria by a lymphoplasmacytic infiltrate, thinning of the muscularis mucosa, and regenerative changes in the crypts including increased number and branching of crypts and increased mitotic activity. These features were absent in the mother-reared control animals, and attenuated in the group treated with LPS plus LGG (Figures 2D-2F). The physical damage in the intestinal musoca of the LPS/LGG group was reduced to a level similar to that of mother's milk-fed rat pups. In the latter intestines, the metaplastic change in the villi was intermediate between that seen in tissues from the control and LPS groups. Notably, this seems to occur as a spectrum, as illustrated by the metaplasia seen in the villi, which approximates that of the LPS-treated group.

### Example 4

This example illustrates the effect of LGG on CINC-1. Small intestine and plasma CINC-1 levels were determined by TiterZyme Enzyme Immunometric Assay kits for rat growth-related oncogene/CINC-1 (Assay Designs, Ann Arbor, MI). Absorbance was determined at 450 nm, and concentration was calculated using the equation derived from a linear standard curve.

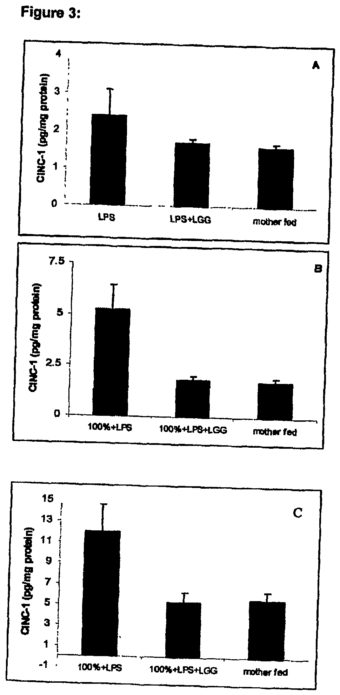

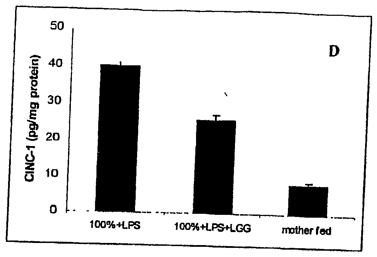

To further investigate the effects of the diets on CINC-1 peptide, CINC-1 production was evaluated by ELISA in the small intestine, liver, lung and plasma. In an initial experiment, LPS administration to rat pups caused an approximate 4-fold elevation in CINC-1 over non-LPS treated pups fed by gastrostomy (data not shown). As shown in Figure 3A, when comparing gastrostomy-fed LPS treated pups to LPS/LGG-treated and mother-fed rats, intestinal CINC-1 levels in pups did not differ significantly among the 3 groups, but suggested a slight trend toward being higher in the group treated with LPS and no LGG. Liver (Fig. 3B) and plasma (Fig. 3C) CINC-1 concentrations, however, were almost 2 fold as high in the LPS treated group that did not receive LGG, but this was significantly attenuated with LGG. The lung (Fig. 3D), here used to determine whether the probiotic effect could extend to a distal organ, also showed a significant elevation (approximately 4 fold) of CINC-1 with LPS treatment when compared to mother-fed controls, but this was significantly attenuated with LGG. In the liver and plasma, LGG supplementation reduced CINC-1 levels to a level which was very similar to that of mother's milk-fed rat pups. These results show that LGG has the ability to reduce systemic inflammation in a formula-fed infant to a level which is similar to that of a breast-fed infant.

### Example 5

This example illustrates the effect of LGG on TNF-α levels in infant rats. Small intestine and plasma TNF-α levels were determined by TiterZyme Enzyme Immunometric Assay kits for TNF-α (Assay Designs, Ann Arbor, MI). Absorbance was determined at 450 nm, and concentration was calculated using the equation derived from a linear standard curve.

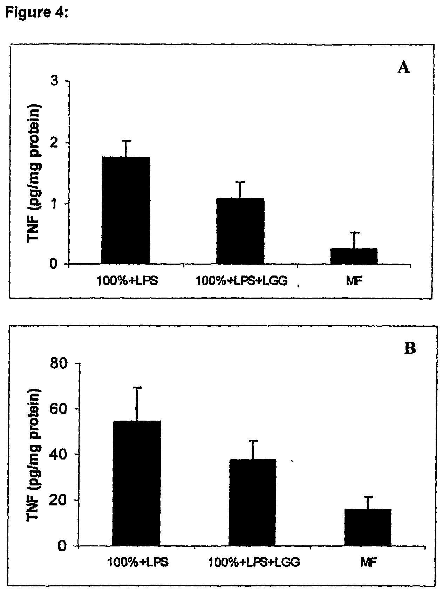

To further investigate the effects of the diets on TNF-α, TNF-α production was evaluated by ELISA in the plasma and lung. Figure 4 illustrates the effect of LGG on TNF-α production from plasma (Fig. 4A) and lung (Fig. 4B) using ELISA. Figure 4 indicates that TNF-α levels were significantly higher in gastrostomy-fed, LPS-treated pups than in mother reared pups and LGG significantly blunted the LPS induced elevation of TNF-α in both the plasma and the lung.

### Example 6

This example illustrates the effect of LGG on MPO levels. MPO activity, a measure of neutrophil accumulation and a marker of tissue injury, was determined by a standard enzymatic procedure. Intestine samples were homogenized on ice in 0.01 M KH₂PO₄ buffer. After centrifugation at 10,000 g for 20 minutes at 4°C, the pellets were resuspended by sonication in cetyltrimethylammonium bromide buffer (13.7 mM CTAB, 50 mM KH₂PO₄, and 50 mM acetic acid, pH 6.0). The supernatant was kept for ELISA analysis. The suspension was centrifuged again at 10,000 g for 15 minutes. The supernatant was then incubated in a 60°C water bath for 2 hours. MPO concentration of the supernatant was measured by the H₂O₂-dependent oxidation of tetramethylbenzidine. Absorbance was determined at 650 nm and compared with a linear standard curve. Protein was measured using the BioRad Dc Protein Assay (BioRad).

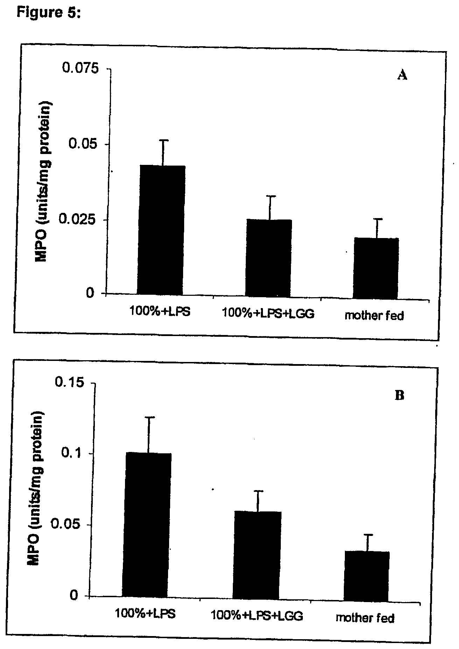

Figure 5 illustrates the effect of LGG on MPO activity in the distal small intestine (Fig. 5A) and lung (Fig. 5B). MPO levels were significantly higher in gastrostomy-fed, LPS-treated pups than in mother reared pups and LGG significantly blunted the LPS induced elevation of MPO in both the distal small intestine and lung. The reduced MPO levels in the LPS/LGG treated rats are very similar to those of the mother-fed rats, showing that LGG reduces systemic inflammation in formula-fed infants to a level which is similar to the level of breast-fed infants.

### Example 7

This example illustrates the effect of LGG on various cytokine levels. Multiplex bead kits were purchased from LINCO Research, Inc. (St. Charles, MO, USA). Cytokines/chemokines were analyzed by a kit that included: granulocyte-macrophage colony-stimulating factor (GMCSF), interferon-A (IFN- λ), interleukin-1α (IL-1α), IL-1α, IL-2, IL-4, IL-5, IL-6, IL-10, IL-12p70, IL-18, Monocyte Chemoattractant protein-1 (MCP-1), GRO/KC (rat CINC-1), and tumor necrosis factor-α (TNF-α). The multiplex assay was performed according to the manufacturer's specifications. Standard curves for each cytokine/chemokine were generated by using the reference concentrations supplied by the manufacturers. Raw data (mean fluorescent intensity) were analyzed by MasterPlex Quantitation Software (MiraiBio, Inc., Alameda, CA, USA) to obtain concentration values.

To further investigate the effects of LPS and LGG on cytokines, 14 cytokines/chemokines from lung, liver, plasma and distal small intestine were analyzed. These included: granulocyte-macrophage colony-stimulating factor (GMCSF), interferon-A (IFN-λ), interleukin-1α (IL-1α), IL-1β, IL-2, IL-4, IL-5, IL-6, IL-10, IL-12p70, IL-18, Monocyte Chemoattractant protein-1(MCP-1), GRO/KC (rat CINC-1), and tumor necrosis factor-α (TNF-α).

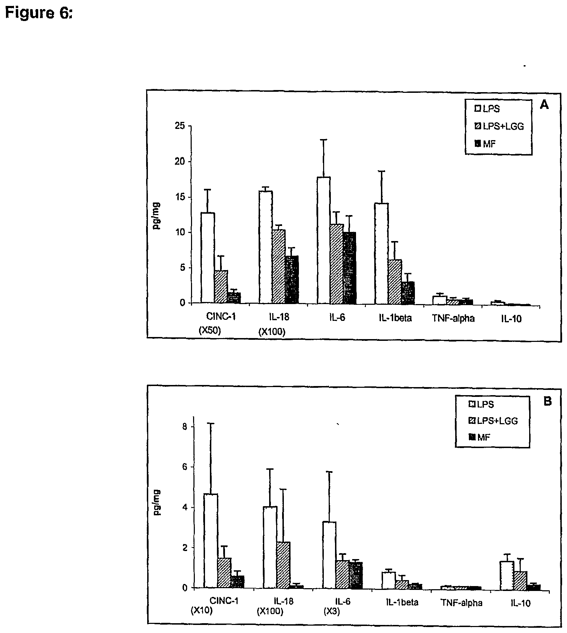

Figure 6A illustrates that lung IL-1β, IL-6, IL-18, GRO/KC (rat CINC-1), and TNF-α were significantly higher in gastrostomy-fed, LPS-treated pups than in mother reared pups and LGG significantly blunted the LPS induced elevation of IL-1β, IL-6, IL-10, IL-18, GRO/KC (rat CINC-1), and TNF-α. Figured 6B shows that liver IL-1β, IL-6, IL-18, and GRO/KC (rat CINC-1) levels were significantly higher in gastrostomy-fed, LPS-treated pups than in mother reared pups and LGG significantly blunted the LPS induced elevation of those cytokines/chemokines. Figure 6C also shows a significant increase of cytokine/chemokine levels in the plasma of the animals that received LPS. This effect was blunted in the animals receiving the LGG.

These results show that LGG supplementation in formula-fed infants reduces systemic inflammation. Further, the results show that LGG reduces systemic inflammation in formula-fed infants to a level which is similar to that of breast-fed infants. This is illustrated in the results described herein through comparison of the LGG-treated group and the group exclusively fed mother's milk. In several instances, administration of LGG results in a particular inflammatory response being not significantly different between the LGG-treated group and the mother's milk-fed group, indicating a similar inflammatory response.

The invention reduces inflammation in the gastrointestinal tract, liver, plasma, lungs, and brain and prevents or reduces physical damage in the intestinal mucosa of a formula-fed infant. As the present invention may be used to improve the inflammatory condition in a infant, it may also prevent the onset of deleterious infections or illnesses.

## Claims

1. Use of *Lactobacillus rhamnosus* GG in combination with at least one long-chain polyunsaturated fatty acid for the manufacture of an infant formula for preventing or reducing systemic inflammation in a formula-fed infant, by administering to the infant an effective amount of *Lactobacillus rhamnosus* GG in combination with at least one long-chain polyunsaturated fatty acid.

2. The use according to claim 1, wherein the long-chain polyunsaturated fatty acid comprises docosahexaenoic acid or arachidonic acid.

3. The use according to claim 2, wherein the long-chain polyunsaturated fatty acid comprises docosahexaenoic acid in an amount of between 3 mg per kg of body weight per day to 150 mg per kg of body weight per day.

4. The use according to claim 2, wherein the long-chain polyunsaturated fatty acid comprises arachidonic acid in an amount of between 5 mg per kg of body weight per day to 150 mg per kg of body weight per day.

5. The use according to claim 1, wherein the long-chain polyunsaturated fatty acid comprises docosahexaenoic acid and arachidonic acid.

6. The use according to claim 1, wherein the systemic inflammation is prevented or reduced in the liver, plasma, and lungs of the infant.

7. The use according to claim 1, wherein the *Lactobacillus rhamnosus* GG and the long-chain polyunsaturated fatty acid are consumed by the infant.

8. The use according to claim 1, wherein the effective amount of *Lactobacillus rhamnosus* GG is between about 1x10⁴ and 1x10¹⁰ cfu/L/kg/day.

9. The use according to claim 1, wherein the effective amount of *Lactobacillus rhamnosus* GG is between about 1x10⁶ and 1x10⁹ cfu/L/kg/day.

10. The use according to claim 1, wherein the effective amount of *Lactobacillus rhamnosus* GG is about 1x10⁸ cfu/Likg/day.

## Patentansprüche

1. Verwendung von *Lactobacillus rhamnosus* GG in Kombination mit mindestens einer langkettigen mehrfach ungesättigten Fettsäure für die Herstellung einer Säuglingsanfangsmilch zur Prävention oder Reduktion von systemischen Entzündungen bei einem mit Anfangsmilch ernährten Säugling durch Verabreichen einer wirksamen Menge von *Lactobacillus rhamnosus* GG in Kombination mit mindestens einer langkettigen mehrfach ungesättigten Fettsäure an den Säugling.

2. Verwendung nach Anspruch 1, wobei die langkettige mehrfach ungesättigte Fettsäure Docosahexaensäure oder Arachidonsäure umfasst.

3. Verwendung nach Anspruch 2, wobei die langkettige mehrfach ungesättigte Fettsäure Docosahexaensäure in einer Menge zwischen 3 mg pro kg Körpergewicht pro Tag und 150 mg pro kg Körpergewicht pro Tag umfasst.

4. Verwendung nach Anspruch 2, wobei die langkettige mehrfach ungesättigte Fettsäure Arachidonsäure in einer Menge zwischen 5 mg pro kg Körpergewicht pro Tag und 150 mg pro kg Körpergewicht pro Tag umfasst.

5. Verwendung nach Anspruch 1, wobei die langkettige mehrfach ungesättigte Fettsäure Docosahexaensäure und Arachidonsäure umfasst.

6. Verwendung nach Anspruch 1, wobei die systemische Entzündung in der Leber, im Plasma und in den Lungen des Säuglings verhindert oder reduziert wird.

7. Verwendung nach Anspruch 1, wobei der *Lactobacillus rhamnosus* GG und die langkettige mehrfach ungesättigte Fettsäure vom Säugling aufgenommen werden.

8. Verwendung nach Anspruch 1, wobei die wirksame Menge von *Lactobacillus rhamnosus* GG zwischen etwa 1 × 10⁴ und 1 × 10¹⁰ KBE/1/kg/Tag beträgt.

9. Verwendung nach Anspruch 1, wobei die wirksame Menge von *Lactobacillus rhamnosus* GG zwischen etwa 1 × 10⁶ und 1 × 10⁹ KBE/1/kg/Tag beträgt.

10. Verwendung nach Anspruch 1, wobei die wirksame Menge von *Lactobacillus rhamnosus* GG etwa 1 × 10⁸ KBE/l/kg/Tag beträgt.

## Revendications

1. Utilisation de *Lactobacillus rhamnosus* GG en association avec au moins un acide gras polyinsaturé à longue chaîne pour la fabrication d'un lait maternisé destiné à la prévention ou à l'atténuation d'une inflammation systémique chez un nourrisson nourri au lait maternisé, en lui administrant une quantité efficace de *Lactobacillus rhamnosus* GG en association avec au moins un acide gras polyinsaturé à longue chaîne.

2. Utilisation selon la revendication 1, dans laquelle l'acide gras polyinsaturé à longue chaîne comprend de l'acide docosahexaénoïque ou de l'acide arachidonique.

3. Utilisation selon la revendication 2, dans laquelle l'acide gras polyinsaturé à longue chaîne comprend de l'acide docosahexaénoïque en une quantité comprise entre 3 mg par kg de poids corporel par jour et 150 mg par kg de poids corporel par jour.

4. Utilisation selon la revendication 2, dans laquelle l'acide gras polyinsaturé à longue chaîne comprend de l'acide arachidonique en une quantité comprise entre 5 mg par kg de poids corporel par jour et 150 mg par kg de poids corporel par jour.

5. Utilisation selon la revendication 1, dans laquelle l'acide gras polyinsaturé à longue chaîne comprend de l'acide docosahexaénoïque et de l'acide arachidonique.

6. Utilisation selon la revendication 1, dans laquelle l'inflammation systémique est prévenue ou atténuée dans le foie, le plasma et les poumons du nourrisson.

7. Utilisation selon la revendication 1, dans laquelle *Lactobacillus rhamnosus* GG et l'acide gras polyinsaturé à longue chaîne sont consommés par le nourrisson.

8. Utilisation selon la revendication 1, dans laquelle la quantité efficace de *Lactobacillus rhamnosus* GG est comprise entre environ 1x10⁴ et 1x10¹⁰ ufc/l/kg/jour.

9. Utilisation selon la revendication 1, dans laquelle la quantité efficace de *Lactobacillus rhamnosus* GG est comprise entre environ 1x10⁶ et 1x10⁹ ufc/l/kg/jour.

10. Utilisation selon la revendication 1, dans laquelle la quantité efficace de *Lactobacillus rhamnosus* GG est d'environ 1x10⁸ ufc/l/kg/jour.
